# EUROPÄISCHE PATENTANMELDUNG

(11) **EP 3 248 552 A1**
(43) Veröffentlichungstag der Anmeldung: **29.11.2017**
(21) Anmeldenummer: 16171293.0
(22) Anmeldetag: 25.05.2016
(51) Int. Cl.: A61B 17/16

(54) **WERKZEUG ZUM ERZEUGEN EINER AUSSPARUNG IN KNOCHENGEWEBE**

(71) Anmelder: WALDEMAR LINK GmbH & Co. KG, 22339 Hamburg (DE)
(72) Erfinder: DMUSCHEWSKY, Klaus, 22397 Hamburg (DE)
(74) Vertreter: Hoffmann Eitle

(57) **Zusammenfassung**

Die vorliegende Erfindung stellt ein Werkzeug zum Erzeugen einer Aussparung in Knochengewebe für ein Implantat bereit, wobei das Werkzeug (1) ein erstes längliches Werkzeugsegment (10) mit einer ersten Längsachse (L1) und einer Aufnahme (14) aufweist, wobei die Aufnahme um die erste Längsachse eine im Wesentlichen radial nach außen gerichtete erste Funktionsfläche (15) und eine radial nach innen gerichtete zweite Funktionsfläche (16, 17, 18) aufweist. Weiterhin weist das Werkzeug ein zweites längliches Werkzeugsegment (20) mit einer zweiten Längsachse (L2) und einem Einführabschnitt (24) auf, der in die Aufnahme des ersten Werkzeugsegments einführbar ist, wobei der Einführabschnitt um die zweite Längsachse mindestens eine radial nach außen gerichtete Funktionsfläche (25) aufweist, die mit der nach außen gerichteten ersten Funktionsfläche des ersten Werkzeugsegments zusammenwirkt. Zudem wirkt eine zweite Funktionsfläche (26, 27, 28) des Einführabschnitts mit der zweiten Funktionsfläche des ersten Werkzeugsegments zusammen. Dabei steht eines der zusammenwirkenden Funktionsflächenpaare (16, 26; 17, 27; 18, 28) für die Übertragung eines Drehmoments miteinander im Eingriff und das andere der zusammenwirkenden Funktionsflächenpaare (15, 25) lässt zwischen sich eine Relativbewegung zu.

## Beschreibung

### TECHNISCHES GEBIET

Die vorliegende Erfindung betrifft ein Werkzeug zum Vorbereiten eines Implantationsorts für ein Implantat und insbesondere zum Erzeugen einer Aussparung im Knochengewebe eines Röhrenknochens. Weiterhin betrifft die vorliegende Erfindung einen Satz aus einem Implantat und dem Werkzeug.

### STAND DER TECHNIK

Bei der Verankerung eines Implantats in einem Röhrenknochen kann zur Führung und unter Umständen auch zur Stabilisierung des Implantats der in der Diaphyse des Röhrenknochens vorhandene Markkanal genutzt werden. Dies hat den Vorteil, dass weniger Knochengewebe zu entfernen ist, um die Öffnung bzw. die Aussparung in dem Knochen für die Aufnahme des Implantats zu erzeugen. Dementsprechend wird im Allgemeinen bei der Verankerung eines Implantats in einem Röhrenknochen angestrebt, das Implantat so in den Markkanal einzuführen, dass der Knochenstock soweit wie möglich bewahrt wird. Dies ist allerdings, wie nachfolgend beschrieben, mit den im Stand der Technik zur Verfügung stehenden Werkzeugen und Techniken nur eingeschränkt möglich.

Für die Präparation des Knochens werden insbesondere zwei Techniken eingesetzt. Eine Technik ist die Verwendung einer Reibahle, die von einem Ende des Röhrenknochens eingeführt wird und durch Rotation um ihre Längsachse eine der Reibahle entsprechende rotationssymmetrische Öffnung in dem Knochen erzeugt. Dabei kann der Knochen vor dem Einführen der Reibahle mit einem Bohrer vorgebohrt worden sein. Diese Technik erlaubt eine schnelle und formgenaue Erzeugung der zum Einsetzen des Implantats erforderlichen Aussparung.

Eine weitere Technik ist die Verwendung einer Handraspel. Mit so einer Raspel kann im Gegensatz zur Reibahle eine nicht rotationssymmetrische Aussparung des Knochens erzeugt werden. Die Raspel wird zur Erzeugung einer Schnittbewegung linear entlang eines Bewegungspfads bewegt. Folglich unterscheidet sich ihre Schnittbewegung grundlegend von der rotatorischen Schnittbewegung der Reibahle. Genauer gesagt fallen bei ihr die Vorschubbewegung und die Schnittbewegung zusammen. Bei einer Reibahle ist die Schnittbewegung hingegen in eine lineare Vorschubbewegung und eine rotatorische Schnittbewegung getrennt.

Als Folge hiervon ist es möglich, mit einer Raspel eine Aussparung im Knochengewebe zu erzeugen, die ein Implantat mit einer Krümmung in Längsrichtung aufnehmen kann. Eine solche Krümmung kann vorgesehen sein, um das Implantat an die Geometrie des jeweiligen Knochens anzupassen. Derartige Implantate werden auch als anatomisch angepasst bezeichnet.

Allerdings hat diese Raspeltechnik den Nachteil, dass beim Abtragen des Knochengewebes nicht die gleiche Formgenauigkeit erreicht werden kann wie bei der vorgenannten Reibahle. Ursache hierfür ist vor allem das Ausführen der Schnitt- und Vorschubbewegung per Hand entlang eines gekrümmten Bewegungspfads, während die rotatorische Schnittbewegung der Reibahle im Allgemeinen über einen Antrieb ausgeführt wird und lediglich die geradlinige Vorschubrichtung manuell per Hand geführt wird. Infolgedessen ist es für den Chirurgen leichter, eine Reibahle zu führen, da er nur die Vorschubbewegung steuern muss, die Schnittbewegung hingegen durch die Rotation der Reibahle ausgeführt wird.

Ungeachtet dessen muss auch mit einer Raspel vor allem bei Implantaten, die mit Hilfe von Knocheneinwuchs verankert werden, eine möglichst passgenaue Aussparung erzeugt werden, um ausreichend Primärstabilität durch Einpressen des Implantats in den Knochen zu erreichen. Damit die im Knochen erzeugte Aussparung möglichst wenig von der vorgesehenen Form abweicht, nimmt der Querschnitt der Raspel von der führenden vorderen Seite nach hinten in der Regel wesentlich stärker zu als der einer Reibahle. Hierdurch wird erreicht, dass durch die Bewegung der Raspel die Aussparung bei jedem Arbeitshub über ihre ganze Länge vergrößert wird. Mit anderen Worten muss der Chirurg durch diese Gestaltung der Raspel weniger darauf achten, dass er dem zuvor eingeschlagenen Bewegungsvektor genau folgt, da über die gesamte Länge der Raspel Material abgetragen wird und so bei jeder Vorschubbewegung eine gewisse Korrektur möglich ist.

Im Gegensatz dazu kann bei einer Reibahle der Querschnitt der Aussparung konstant gehalten werden, obwohl die Reibahle relativ zur Vorschubrichtung weiter vorne damit fortfährt, Knochengewebe zu entfernen. Als Ergebnis kann die Auslegung des Implantats in Bezug auf die Materialeigenschaften funktionell besser angepasst werden.

Beide Techniken haben den Nachteil, dass sie intraoperativ nicht in der Lage sind, auf die individuelle Anatomie des jeweiligen Röhrenknochens zu reagieren. Die Folge sind häufig ungewünschte Einschnitte in das Knochengewebe. Dies ist vor allem bei Verletzungen des kortikalen Knochengewebes im Bereich der Diaphyse von Nachteil. Der Röhrenknochen wird infolgedessen in diesem Bereich strukturell geschwächt. Zudem macht die hervorgerufene Kerbwirkung den Knochen anfällig für Biegebelastungen und kann schlimmstenfalls zu einem Knochenbruch führen.

Weiterhin kann an dem distalen Ende des Implantatschafts, der lediglich durch den Markraum geführt, nicht aber abgestützt werden soll, ein sogenanntes Stress-Shielding verursacht werden, d. h. das Implantat stützt sich an dem den Markraum umschließenden kortikalen Gewebe ab. Es kommt zu einer Fehlbelastung des Knochens durch eine Krafteinleitung im Bereich der Diaphyse. Das Knochengewebe reagiert hierauf mit einem Aufbau von Knochengewebe im Bereich der unnatürlichen Krafteinleitung und mit Knochenresorption an der Stelle, wo die Krafteinleitung eigentlich erfolgen sollte. Dieses Phänomen ist vor allem bei Gelenkendoprothesen bekannt und kann zu einem Versagen des Knochengewebes und des Implantats bzw. der Prothese führen.

### ZUSAMMENFASSUNG DER ERFINDUNG

Ziel war es folglich, ein Werkzeug zur Öffnung eines Röhrenknochens für ein Implantat bereitzustellen, wobei das Werkzeug imstande sein soll, auf die anatomischen Gegebenheiten eines Knochens, insbesondere eines Röhrenknochens, zu reagieren. Gleichzeitig soll die Resektion von Knochengewebe möglichst gering gehalten werden. Mit anderen Worten sollte das Werkzeug der vorliegenden Erfindung erreichen, einen Implantationsort möglichst knochenschonend vorzubereiten und gleichzeitig die Versorgung mit einem anatomisch angepassten und dennoch knochenschonendem Implantat zu ermöglichen.

In Anbetracht dieser Herausforderung stellt die vorliegende Erfindung ein Werkzeug zum Erzeugen einer Aussparung in Knochengewebe für ein Implantat bereit, wobei das Werkzeug ein erstes längliches Werkzeugsegment mit einer ersten Längsachse und einer Aufnahme aufweist. Die Aufnahme weist um die erste Längsachse eine im Wesentlichen radial nach außen gerichtete erste Funktionsfläche und eine radial nach innen gerichtete zweite Funktionsfläche auf. Weiterhin weist das Werkzeug ein zweites längliches Werkzeugsegment mit einer zweiten Längsachse und einem Einführabschnitt auf, der in die Aufnahme des ersten Werkzeugsegments einführbar ist, wobei an dem Einführabschnitt um die zweite Längsachse mindestens eine radial nach außen gerichtete Funktionsfläche ausgebildet ist, die mit der nach außen gerichteten ersten Funktionsfläche des ersten Werkzeugsegments zusammenwirkt. Zudem wirkt eine der mindestens einen Funktionsfläche des Einführabschnitts mit der zweiten Funktionsfläche des ersten Werkzeugsegments zusammen. Dabei steht eines der zusammenwirkenden Funktionsflächenpaare für die Übertragung eines Drehmoments miteinander im Eingriff und das andere der zusammenwirkenden Funktionsflächenpaare lässt zwischen sich eine Relativbewegung zu.

Ein derartiges zweigeteiltes Werkzeug hat den Vorteil, dass es sich beim Erzeugen der vorgegebenen Aussparung für eine Prothese an die jeweilige anatomische Situation des Patienten, wie im Folgenden beschrieben, in einem gewissen Umfang anpassen kann.

Der Einführabschnitt ist zwischen einer radial nach innen gerichteten Funktionsfläche und einer radial nach außen gerichteten Funktionsfläche der Aufnahme zu der ersten Längsachse radial versetzt aufgenommen. Zudem schneiden sich die Längsachsen der beiden Werkzeugsegmente in einem Winkel. In dem in die Aufnahme eingeführten Zustand kann sich die radial nach außen gerichtete Funktionsfläche des zweiten Werkzeugsegments aufgrund dieses Aufbaus um die radial nach außen gerichtete erste Funktionsfläche des ersten Werkzeugsegments bewegen, ohne dass dabei eine Drehung zwischen den Längsachsen der beiden Werkzeugsegmente übertragen wird. Das heißt, dass wenn das erste oder zweite Werkzeugsegment festgehalten wird, ein Ende des Einführabschnitts genauso wie das diesem Ende gegenüberliegende andere Ende auf einer umlaufenden Bahn bewegbar ist. Mit anderen Worten dreht sich eines der Werkzeugsegmente um die Längsachse des anderen Werkzeugsegments.

Wird hingegen das in Vorschubrichtung gesehene vordere Werkzeugsegment geführt und das andere Werkzeugsegment gedreht, ist es möglich, über das in Eingriff stehende Funktionsflächenpaar eine Drehbewegung zwischen den beiden Längsachsen zu übertragen. Das eine Relativbewegung zulassende Funktionsflächenpaar steht miteinander in Kontakt und stützt so den Einführabschnitt in der Aufnahme ab. Mit anderen Worten gleiten die beiden Funktionsflächen relativ zueinander. Hierdurch bleiben die beiden winklig zueinander ausgerichteten Längsachsen in derselben absoluten Ebene und es werden eine Drehung und ein Drehmoment von der einen auf die andere Längsachse übertragen. Als Folge hiervon entsteht eine Aussparung im Knochengewebe, die mit der Außengeometrie des Werkzeugs korrespondiert und in der ein entsprechendes Implantat verankerbar ist.

Durch diese Eigenschaften der Werkzeugsegmente kann das vordere Werkzeugsegment beim Einführen in das Knochengewebe dem Weg des geringsten Widerstands folgen. Mit anderen Worten beeinflusst das Knochengewebe durch seine Dichte und Struktur den Vorschubpfad. Als Folge dieser "Führung" kann es zu einer veränderten Ausrichtung des Werkzeugs durch Drehung des einen Werkzeugsegments um die Längsachse des anderen Werkzeugsegments kommen. Dabei erfolgt keine oder nur eine teilweise Übertragung der Drehbewegung und des Drehmoments von einer Längsachse zur anderen Längsachse, während ein anderer Teil eine Drehung des anderen Werkzeugsegments um diese eine Längsachse verursacht. Hierdurch kann insbesondere das kortikale Knochengewebe der Diaphyse eines Röhrenknochens geschont werden, da sich das vordere Werkzeugsegment beim Einführen in den Markkanal in einem gewissen Ausmaß selbstständig entlang dieses Markkanals ausrichtet, dabei aber die grundlegende Geometrie der Aussparung für das Implantat beibehalten wird. Mit anderen Worten schont das Werkzeug das umliegende Knochengewebe, indem es bei Erzeugung der Aussparung so wenig Knochengewebe wie möglich bzw. zunächst das Knochengewebe mit einer geringeren Dichte entfernt.

Im Ergebnis ruft die Anordnung der beiden Werkzeugsegmente in einem Winkel eine vorbestimmte Aussparung bzw. Öffnung für einen Implantatschaft in dem Knochen hervor, die einen entsprechenden Winkel aufweist. Damit verbindet das erfindungsgemäße Werkzeug die Vorteile der zuvor genannten beiden Techniken, indem der Chirurg lediglich die Vorschubbewegung ausführen muss, während die rotatorische Schnittbewegung durch einen Antrieb erfolgen kann, und gleichzeitig die Implantation eines Implantats ermöglicht wird, das anatomisch angepasster ist.

Bei einer weiteren Ausführungsform des Werkzeugs wird die erste Funktionsfläche des ersten Werkzeugsegments durch einen Vorsprung in der Aufnahme ausgebildet, wobei der Vorsprung vorzugsweise konisch oder zylindrisch ist.

Durch diese Ausführung der ersten Funktionsfläche des ersten Werkzeugsegments wird insbesondere das Einführen des Einführabschnitts des zweiten Werkzeugsegments erleichtert.

Dabei handelt es sich bei der ersten Funktionsfläche des ersten Werkzeugsegments vorzugsweise um eine konische Fläche, insbesondere um eine konische Fläche mit einem kreisförmigen Querschnitt. Der Vorsprung verjüngt sich vorzugsweise in Richtung der Aufnahmeöffnung. Handelt es sich bei dem Vorsprung um einen zylindrischen Vorsprung, so weist auch dieser vorzugsweise einen Kreisquerschnitt auf.

Bei einer besonders bevorzugten Ausführungsform des Werkzeugs steht das in Eingriff stehende Funktionsflächenpaar für die Drehmomentübertragung formschlüssig im Eingriff.

Die Verwendung eines Formschlusses im Vergleich zu einem Reibschluss für die Drehmomentübertragung hat den Vorteil einer definierten Kinematik, bei welcher der Gefahr eines Durchrutschens zwischen den Körpern vorgebeugt ist.

Bei einer weiteren bevorzugten Ausführungsform des Werkzeugs weisen die Querschnitte der Funktionsflächen der Funktionsflächenpaare senkrecht zu der jeweiligen Längsachse einen im Wesentlichen kreisförmigen Umfang auf.

Diese Ausführungsform führt dazu, dass das erste und zweite Werkzeugsegment in einem konstanten Winkel relativ zueinander angeordnet sind, sodass sich der Einführabschnitt des zweiten Werkzeugsegments in der Aufnahme im Wesentlichen in einer zu der Längsachse des ersten Werkzeugsegments senkrechten Ebene auf einer Kreisbahn um diese Längsachse bewegt.

Durch den konstanten Winkel kann die Vorschubbewegung insbesondere per Hand durch den Chirurgen unter genauer Kontrolle erfolgen. Dabei entspricht die hierdurch entstehende Aussparung im Knochengewebe den in das Knochengewebe eingeführten Werkzeugsegmenten und damit der für das Implantat vorgesehenen vorbestimmten Geometrie und Abmessung.

Unter einem im Wesentlichen kreisförmigen Umfang ist in diesem Zusammenhang auch ein Umfang zu verstehen, der regelmäßig von einer perfekten Kreisform abweichen kann. Dies ist beispielsweise der Fall, wenn das betreffende Funktionsflächenpaar ein Drehmoment mit Hilfe eines formschlüssigen Eingriffs überträgt. Um einen solchen zu erreichen, müssen am Umfang der Funktionsflächen Aussparungen bzw. Vorsprünge mit einer Form vorgesehen sein, mit der ein Eingriff zwischen den Funktionsflächen ermöglicht wird.

Bei einer weiteren besonders bevorzugten Ausführungsform weisen das erste Werkzeugsegment und das zweite Werkzeugsegment mindestens zwei getrennte zwischen ihnen zusammenwirkende Funktionsflächenpaare auf.

Dadurch, dass bei dieser Ausführungsform jedes Funktionsflächenpaar jeweils zwei eigene Funktionsflächen aufweist, kann das Werkzeug mit einem geringeren Durchmesser versehen werden als ein Werkzeug, bei dem eine Funktionsfläche in einem Bereich mit einer Funktionsfläche zur Übertragung eines Drehmoments im Eingriff ist und in einem anderen Bereich, in dem eine Relativbewegung ausgeführt wird, mit einer weiteren Funktionsfläche in Kontakt ist.

Bei einer weiteren Ausführungsform weisen das erste Werkzeugsegment und das zweite Werkzeugsegment mindestens drei getrennte, zwischen ihnen zusammenwirkende Funktionsflächenpaare auf. Dabei lässt das erste Funktionsflächenpaar zwischen sich eine Relativbewegung zu, das zweite Funktionsflächenpaar unterstützt den Einführabschnitt in der Aufnahme und das Drehmoment ist durch das dritte Funktionsflächenpaar zwischen dem ersten und dem zweiten Werkzeugsegment übertragbar.

Bei dieser Ausführungsform hat diese funktionelle Aufteilung, wie nachfolgend erläutert, den Vorteil eines sehr genauen Bewegungsablaufs. Wie oben beschrieben, liegt der Einführabschnitt auf der einen Seite einer radial nach innen gerichteten Funktionsfläche der Aufnahme gegenüber und auf der anderen Seite einer radial nach außen gerichteten Funktionsfläche der Aufnahme. Dabei wirken das Funktionsflächenpaar für die Übertragung des Drehmoments und das Funktionsflächenpaar zur Lagerung auf der gleichen Seite, während das Funktionsflächenpaar, dass eine Relativbewegung zulässt, auf der anderen Seite wirkt. Da Drehmomentübertragung und Abstützung bzw. Lagerung auf derselben Seite stattfinden, rollt das unterstützende Funktionsflächenpaar folglich aufeinander ab. Dies bedeutet auch, dass das miteinander in Kontakt stehende Funktionsflächenpaar, welches eine Relativbewegung zulässt, ebenfalls eine unterstützende Funktion aufweist.

Weiterhin hat diese Ausführungsform den Vorteil, dass die Übertragung eines Drehmoments über den gesamten Umfang zwischen der Aufnahme und dem Einführabschnitt ermöglicht wird, da das zweite Funktionsflächenpaar die Stützfunktion übernimmt.

Bei einer weiteren Ausführungsform ist die radial nach außen gerichtete Funktionsfläche des ersten Werkzeugsegments auswechselbar und vorzugsweise ein Teil eines dritten Werkzeugsegments.

Bei dieser Ausführungsform wird die Anpassungsfähigkeit des Werkzeugs an die Anatomie eines Patienten erhöht, indem durch Auswechseln der ersten Funktionsfläche der Winkel zwischen den Längsachsen des ersten und zweiten Werkzeugsegments verändert werden kann. Diese Eigenschaft des ersten Werkzeugsegments dieser Ausführungsform ergänzt auf vorteilhafte Weise die Austauschbarkeit des zweiten Werkzeugsegments, welches, wie oben dargelegt, über den Einführabschnitt in die Aufnahme des ersten Werkzeugsegments einführbar ist.

Ist die auswechselbare Funktionsfläche Teil eines dritten Werkzeugsegments, so ermöglicht dies, die Aussparung im Knochengewebe in deren proximalem Bereich zusätzlich zu bearbeiten und anzupassen.

Weiterhin kann das Werkzeug auf diese Weise ab einer vorbestimmten Tiefe der Aussparung verlängert werden. Dadurch ist die Länge des Werkzeugs anpassbar, sodass es nahezu unabhängig von der Aussparungstiefe auf einfache Weise geführt werden kann.

Bei einer weiteren Ausführungsform weist mindestens eines der Werkzeugsegmente an einer nach außen gewandten Umfangsfläche mindestens ein Schneidelement auf.

Ein derartiges vorgesehenes Schneidelement ist vor allem dann von Vorteil, wenn ein größeres Knochengewebevolumen zu entfernen ist oder es sich bei dem Knochengewebe um sehr dichtes Gewebe handelt. Letzteres ist zum Beispiel bei sehr dichter Spongiosa und bei Kortikalis der Fall, sodass eine Anpassung der Aussparung im Knochengewebe an eine für das Implantat vorgesehene Geometrie durch Verdichten des Knochengewebes nicht erreichbar ist.

Bei einer besonders bevorzugten Ausführungsform weist die Aufnahme des ersten Werkzeugsegments eine Haltefläche auf, die durch eine innenliegende Querschnittserweiterung der Aufnahme ausgebildet wird und an der sich eine dazu korrespondierende Querschnittserweiterung des Einführabschnitts im eingeführten Zustand abstützt, sodass das zweite Werkzeugsegment im ersten Werkzeugsegment gehalten wird.

Bei dieser Ausführungsform wird das zweite Werkzeugsegment in dem ersten Werkzeugsegment gehalten und ist insoweit gesichert, dass nach Einführen des Einführabschnitts in die Aufnahme des ersten Werkzeugsegments das Werkzeug bis zum Aufsetzen auf das Knochengewebe über die ringförmige Haltefläche gehalten wird.

Durch die Querschnittserweiterung in der Aufnahme des ersten Werkzeugsegments wird eine Hinterschneidung erzeugt, die eine im Wesentlichen zu der Längsachse des ersten Werkzeugsegments senkrecht verlaufende Stufe ausbildet. Auf dieser kann ein Absatz abgestützt werden, der durch die korrespondierende Querschnittserweiterung an dem Einführabschnitt des zweiten Werkzeugsegments vorhanden und von der Spitze des Einführabschnitts abgewandt ist.

Es ist auch möglich, die Haltefläche des ersten Werkzeugsegments als eine umlaufende Nut in der Aufnahme vorzusehen, in die ein damit korrespondierender Vorsprung des Einführabschnitts eingreift. In jedem Fall wird einem einfachen Herausfallen des zweiten Werkzeugsegments aus dem ersten Werkzeugsegment vorgebeugt.

Bei einer weiteren besonders bevorzugten Ausführungsform ist auf der Höhe des Schnittpunkts der ersten Längsachse und der zweiten Längsachse ein Funktionsflächenpaar angeordnet, das eine radial nach außen gerichtete Funktionsfläche des Einführabschnitts und eine radial nach innen gerichtete Funktionsfläche der Aufnahme aufweist, wobei das Funktionsflächenpaar bevorzugt für die Drehmomentübertragung im Eingriff steht.

Durch das Anordnen der Funktionsflächen im Schnittpunkt der beiden Längsachsen erfolgt eine besonders präzise und einfache Führung des zweiten Werkzeugsegments im ersten Werkzeugsegment. Insbesondere können die beiden Funktionsflächen bei dieser Ausführungsform über ihren Umfang in Kontakt stehen, sodass diese Funktionsflächen ausreichen, um die Werkzeugsegmente in Bezug auf ihren rotatorischen Freiheitsgrad bestimmt zu unterstützen. Dies trifft sowohl auf ein Funktionsflächenpaar für eine Drehmomentübertragung als auch ein Funktionsflächenpaar zur Unterstützung bzw. Lagerung oder ein Funktionsflächenpaar zu, dass zwischen sich eine Relativbewegung zulässt.

Bevorzugt ist in diesem Abschnitt allerdings eine Drehmomentübertragung ausführbar, die über mehrere Stellen am Umfang erfolgen kann und bevorzugt bei einem formschlüssigen Eingriff eine Abstützung auf einer in etwa diametral gegenüberliegenden Seite erfolgt. Bei dieser Ausführungsform wird einem Biegemoment um eine Achse senkrecht zu den Längsachsen vorgebeugt, sodass die Belastung auf das Material geringer ist und das Werkzeug kompakter, d.h. mit einem geringeren Durchmesser, ausgeführt werden kann. Es handelt sich also auch bei dieser Ausführungsform um eine Maßnahme, welche eine bessere Führung des Werkzeugs ermöglicht.

Des Weiteren wird ein Werkzeugsegment mit einer Längsachse bereitgestellt, um die zum Einführen eines weiteren Werkzeugsegments eine Aufnahme in dem Werkzeugsegment ausgebildet ist. Die Aufnahme weist eine Öffnung, mindestens eine radial nach innen gerichteten Funktionsfläche und eine Endfläche auf, die zumindest abschnittsweise durch eine radial nach außen gerichtete Funktionsfläche ausgebildet ist, wobei eine der Funktionsflächen für die Übertragung eines Drehmoments zu dem weiteren Werkzeugsegment vorgesehen ist und die andere Funktionsfläche eingerichtet ist, eine Relativbewegung zu dem weiteren Werkzeugsegment zuzulassen.

Dieses Werkzeugsegment weist mit seinen Merkmalen die oben beschriebenen Vorteile auf und bildet zusammen mit einem weiteren Werkzeugsegment ein Werkzeug aus, mit dem eine Aussparung im Knochengewebe zur Aufnahme eines Implantats erzeugt werden kann. Dabei hat die Werkzeugaufnahme den Vorteil, dass sie verschiedene Werkzeuge aufnehmen kann. So ist es beispielsweise möglich, zunächst die Aussparung im Knochengewebe mit einem Bohrer vorzubereiten und anschließend mit einer Reibahle nachzuarbeiten. Dabei ermöglicht es der oben beschriebene strukturelle Aufbau der Aufnahme, dass die Aussparung im Knochengewebe einen winkligen Verlauf aufweist, der einem winkligen Verlauf der Geometrie des einzusetzenden Implantats entspricht.

### KURZE BESCHREIBUNG DER FIGUREN

In den begleitenden Figuren, auf die im Folgenden im Rahmen der ausführlichen Beschreibung bevorzugter Ausführungsformen Bezug genommen wird, werden Elemente mit gleicher Funktion und/oder Gestaltung mit gleichen Bezugszeichen gekennzeichnet. Dabei zeigt
Figur 1 eine schematische Ansicht einer beispielhaften Ausführungsform des erfindungsgemäßen Werkzeugs, welches dreiteilig ausgeführt ist,
Figur 2 eine schematische Schnittansicht einer beispielhaften Ausführungsform des erfindungsgemäßen Werkzeugs, welche die gegenseitige Lagerung zweier Werkzeugsegmente verdeutlicht, und
Figur 3 eine schematische dreidimensionale Seitenansicht einer beispielhaften Ausführungsform des erfindungsgemäßen Werkzeugs zur Verdeutlichung der Kinematik.

### AUSFÜHRLICHE BESCHREIBUNG BEVORZUGTER AUSFÜHRUNGSFORMEN

In der folgenden Beschreibung bezieht sich die Bezeichnung "distal" auf die in Vorschubrichtung des Werkzeugs vorne liegende Seite einer Komponente. Dementsprechend bezieht sich die Bezeichnung "proximal" auf die in Vorschubrichtung des Werkzeugs hinten liegende Seite einer Komponente.

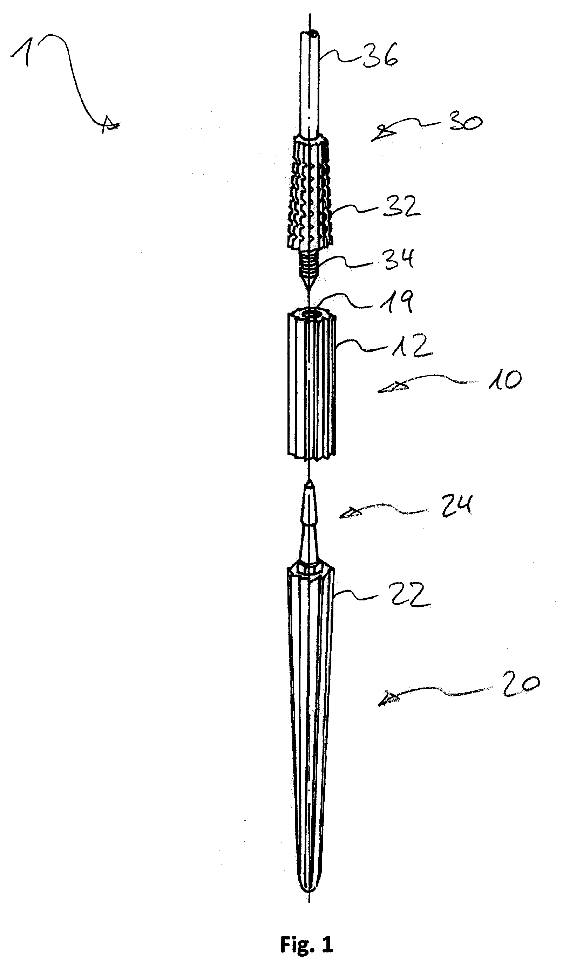

Figur 1 zeigt eine beispielhafte Ausführungsform eines erfindungsgemäßen Werkzeugs 1, mit dem eine Aussparung in einem Röhrenknochen erzeugt werden kann. Das Werkzeug 1 weist ein erstes Werkzeugsegment 10, ein zweites Werkzeugsegment 20 und ein drittes Werkzeugsegment 30 auf.

Das zweite Werkzeugsegment 20 weist Schneidelemente 22 auf, mit denen eine Aussparung bzw. ein Loch in einem Knochen, insbesondere einem Röhrenknochen, erzeugt werden kann. Genauso kann das Werkzeug 1 als Reibahle ausgeführt dafür vorgesehen sein, eine bereits bestehende oder vorbereitete Öffnung im Knochengewebe zu erweitern. Das Werkzeug 1 ist folglich und wie oben bereits beschrieben dazu vorgesehen, eine Aussparung vorzubereiten, die bezüglich ihrer Geometrie und ihren Abmessungen an ein zu implantierendes Implantat angepasst ist.

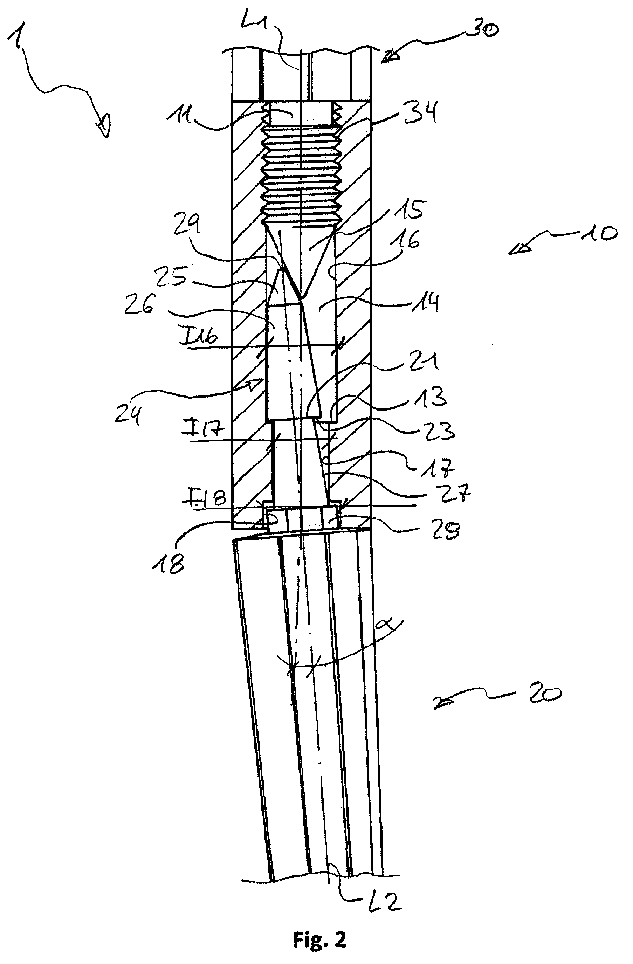

Das in Vorschubrichtung vorne befindliche längliche Werkzeugsegment 20 kann, wie in Figur 2 gezeigt, konisch ausgebildet sein. Genauso ist es möglich, das Werkzeug 1 für die Erzeugung einer zylindrischen Öffnung auszubilden, wie beim ersten Werkzeugsegment 10 der Fall. Für die Erzeugung der Aussparung sind am Umfang des zweiten Werkzeugsegments 20 Schneidelemente 22 vorgesehen. Mit diesen kann mittels einer Rotation des zweiten Werkzeugsegments 20 um seine Längsachse umliegendes Knochengewebe abgetragen werden.

Des Weiteren weist das zweite Werkzeugsegment 20 für eine Verbindung mit dem ersten Werkzeugsegment 10 und zur Übertragung einer Drehung zwischen den beiden Werkzeugsegmenten einen Einführabschnitt 24 auf. Der Einführabschnitts 24 befindet sich in der Vorschubrichtung entlang der Längsachse gesehen am hinteren Ende des zweiten Werkzeugsegments 20.

Der Einführabschnitts 24 ist in eine nicht gezeigte Aufnahme 14 des ersten Werkzeugsegments 10 einführbar. Wie nachfolgend näher beschrieben, sind dabei das erste Werkzeugsegment 10 und das zweite Werkzeugsegment 20 in einem Winkel zueinander angeordnet.

Das erste Werkzeugsegment 10 kann zum Ausbilden einer Aussparung bzw. einer Öffnung im Knochengewebe in der gezeigten Ausführungsform des Werkzeugs 1 ebenfalls am Umfang in Längsrichtung des Werkzeugsegments 10 angeordnete Schneidelemente 12 aufweisen. Wie in Figur 1 an der durch die Schneidelemente 12 ausgebildeten Mantelfläche zu erkennen, ist das Werkzeugsegment 10 zum Ausbilden eines zylindrischen Loches vorgesehen. Selbstverständlich kann auch bei dem ersten Werkzeugsegment 10 zumindest abschnittsweise eine sich verjüngende Ausführungsform vorgesehen sein. Weiterhin ist es möglich, auf dem ersten Werkzeugsegment 10 nur abschnittsweise oder gar keine Schneidelemente 12 vorzusehen.

Auf der in Vorschubrichtung des ersten Werkzeugsegments 10 vorne liegenden Seite ist die Aufnahme 14 (siehe Figur 2) vorgesehen, in die das zweite Werkzeugsegment 20 einsetzbar ist. Auf der in Längsrichtung gegenüberliegenden Seite des ersten Werkzeugsegments 10 ist eine Aufnahme für ein drittes Werkzeugsegment 30 vorgesehen.

Bei der vorliegenden beispielhaften Ausführungsform wird das dritte Werkzeugsegment 30 über einen Vorsprung 11, der ein Gewinde 34 aufweist, mit einem Gewinde 19, vorzugsweise einem Gewindeloch, des ersten Werkzeugsegments 10 lösbar verbunden. Alternativ können auch andere dem Fachmann geläufige Verbindungstechniken für rotierende Werkzeuge verwendet werden. Weiterhin ist es möglich, das erste Werkzeugsegment 10 und das dritte Werkzeugsegment 30 einstückig auszuführen.

Auch das dritte Werkzeugsegment 30 kann, wie in der beispielhaften Ausführungsform der Figur 1 gezeigt, Schneidelemente 32 aufweisen.

An dem distalen Ende des länglichen dritten Werkzeugsegments 30 befindet sich das oben genannte Gewinde 34 für eine Verbindung mit dem ersten Werkzeugsegment 10. An dem gegenüberliegenden oder proximalen Ende des dritten Werkzeugsegments 30 ist ein Einspannelement 36 vorgesehen, das bei der vorliegenden Ausführungsform zylindrisch ausgeführt ist und beispielsweise in einer Bohrmaschine eingespannt werden kann. Ein über das Verbindungselement 36 eingeleitetes Drehmoment kann von dem dritten Werkzeugsegment 30 auf das erste Werkzeugsegment 10 und von diesem wiederum auf das zweite Werkzeugsegment 20 übertragen werden.

Dieser modulare Aufbau des Werkzeugs 1 hat den Vorteil, dass es sehr gut an das zu implantierende Implantat bzw. die hierfür zu schaffenden Öffnung im Knochengewebe angepasst werden kann.

Figur 2 zeigt eine Schnittansicht durch das erste Werkzeugsegment 10, welches an seinem proximalen Ende mit einem dritte Werkzeugsegment 30 und an seinem distalen Ende mit einem zweiten Werkzeugsegment 20 verbunden ist. Bei der veranschaulichten Ausführungsform des ersten Werkzeugsegments 10, weist dieses ein Durchgangsloch auf. Dieses Durchgangsloch ist an seinem proximalen Ende mit einem Gewinde 19 zur Aufnahme eines Gewindeabschnitts 34 an einem distalen Vorsprung des dritten Werkzeugsegments 30 vorgesehen. Der nach dem Einschrauben des Gewindeabschnitts 34 verbleibende Freiraum im Durchgangsloch bildet die Aufnahme 14 für den Einführabschnitt 24 des zweiten Werkzeugsegments 20 aus.

Die Aufnahme 14 weist von proximal nach distal eine radial nach innen gerichtete zweite Funktionsfläche 16, eine ringförmige Haltefläche 13, eine radial nach innen gerichtete dritte Funktionsfläche 17 und eine radial nach innen gerichtete vierte Funktionsfläche 18 auf. Die Funktionsflächen sind wie dargestellt vorzugsweise zylindrisch und noch bevorzugter kreisförmig zylindrisch ausgebildet. Wie weiter unten im Rahmen von Abwandlungen der vorliegenden Ausführungsform beschrieben, können auch weniger Funktionsflächen vorgesehen sein.

Die radial nach innen gerichtete zweite Funktionsfläche 16 weist eine Innenabmessung I16 auf, die größer ist als die Innenabmessung I17 der radial nach innen gerichteten dritten Funktionsfläche 17. Aufgrund der hierdurch entstehenden Erweiterung der Aufnahme 14 in proximaler Richtung wird ein Absatz ausgebildet, der eine ringförmige Haltefläche 13 in dem der Aufnahme 14 des zweiten Werkzeugsegments 20 ausbildet. Die ringförmige Haltefläche 13 ist dabei vorzugsweise senkrecht zur Längsachse L1 des ersten Werkzeugsegments 10 ausgebildet. Die radial nach innen gerichtete vierte Funktionsfläche 18 weist eine Innenabmessung I18 auf, die wiederum größer ist als der Durchmesser I17.

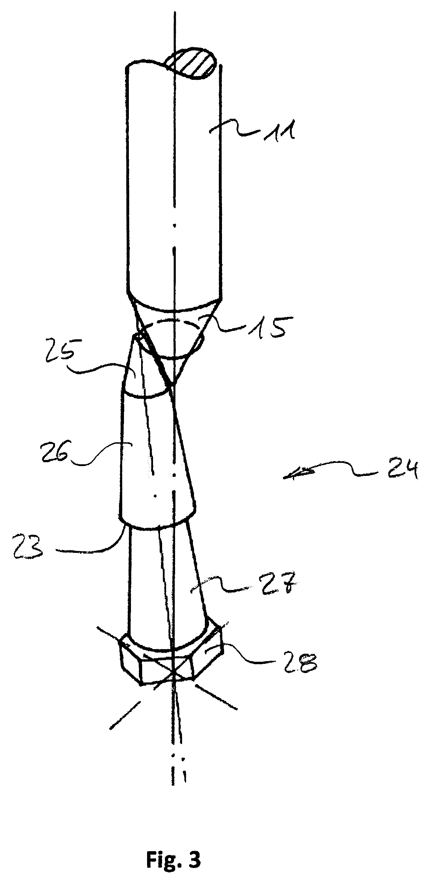

Bei der in Figur 2 veranschaulichten Ausführungsform sind die radial nach innen gerichteten Funktionsflächen 16 und 17 kreisförmig zylindrisch ausgebildet. Folglich handelt es sich bei der Innenabmessung I16 der zweiten Funktionsfläche 16 und der Innenabmessung I17 der dritten Funktionsfläche 17 um Innendurchmesser. Hingegen ist die radial nach innen gerichtete vierte Funktionsfläche 18 des ersten Werkzeugsegments 10 mit einer Geometrie ausgebildet, welche einen Eingriff mit einer korrespondierenden Funktionsfläche 28 des Einführabschnitts 24 zum Übertragen eines Drehmoments ermöglicht. Bei der vorliegenden beispielhaften Ausführungsform in den Figuren 2 und 3 handelt es sich um einen Sechskant. Es ist verständlich, dass jeglicher anderer Mehrkant oder Geometrie verwendet werden kann, solange diese eine Übertragung eines Drehmoments ermöglicht. Dies gilt entsprechend für die radial nach außen gerichtete vierte Funktionsfläche 28 des zweiten Werkzeugsegments 20.

Weiterhin befindet sich in der Aufnahme 14 des ersten Werkzeugsegments 10 eine radial nach außen gerichtete erste Funktionsfläche 15, die beim vorliegenden Ausführungsbeispiel durch den mit einem Gewinde 34 vorgesehenen Vorsprung 11 des dritten Werkzeugsegment 30 ausgebildet wird. Alternativ ist es möglich, die radial nach außen gerichtete erste Funktionsfläche 15 als Teil des ersten Werkzeugsegments 10 auszubilden. Bei dem in Figur 2 veranschaulichten Ausführungsbeispiel ist die Funktionsfläche 15 als Kegelfläche ausgebildet.

Der in die Aufnahme 14 eingeführte Einführabschnitt 24 des zweiten Werkzeugsegments 20 weist von proximal nach distal eine radial nach außen gerichtete erste Funktionsfläche 25, eine radial nach außen gerichtete zweite Funktionsfläche 26, eine radial nach außen gerichtete dritte Funktionsfläche 27 und, wie oben bereits erwähnt, eine radial nach außen gerichtete vierte Funktionsfläche 28 auf.

Die Funktionsflächen 25, 26 und 27 verjüngen sich in dem veranschaulichten Ausführungsbeispiel von distal zum proximalen Endpunkt 29 des Einführabschnitts 24 hin. Die radial nach außen gerichtete erste Funktionsfläche 25 des zweiten Werkzeugsegments 20 ist wie die radial nach außen gerichtete erste Funktionsfläche 15 des ersten Werkzeugsegments 10 kegelförmig ausgebildet. Genauso kann mindestens eine der Funktionsflächen 15 und 25 kegelstumpfartig ausgebildet sein.

Die radial nach außen gerichtete zweite Funktionsfläche 26 schließt sich distal an die radial nach außen gerichtete Funktionsfläche 25 an und ist kegelstumpfförmig ausgebildet. An die Funktionsfläche 26 schließt sich die ebenfalls kegelstumpfförmig ausgebildete Funktionsfläche 27 an. Diese weist an ihrem proximalen Ende einen geringeren Durchmesser auf als die ebenfalls bei diesem proximalen Ende befindliche distale Basis 21 des Kegelstumpfes der Funktionsfläche 26. Hierdurch wird über die Funktionsfläche 26 und die Funktionsfläche 27 ein Absatz ausgebildet. Die Absatzfläche dient als ringförmige Stützfläche 23, die, wie weiter unten beschrieben, mit der Haltefläche 13 des ersten Werkzeugsegments zusammenwirkt.

Wie oben beschrieben, weist das Werkzeug 1 für eine Übertragung eines Drehmoments mindestens ein Funktionsflächenpaar, das zwischen sich eine Relativbewegung zulässt, und mindestens ein Funktionsflächenpaar auf, das formschlüssig oder reibschlüssig im Eingriff steht.

Das im Eingriff stehende Funktionsflächenpaar bildet bei dem Werkzeug die Drehmomentübertragungseinrichtung zur Übertragung eines Drehmoments zwischen den Werkzeugsegmenten aus. Mit Hilfe dieses Drehmoments wird von zumindest einem der Werkzeugsegmente eine Schneidbewegung ausgeführt. Das Funktionsflächenpaar, welches in seinem Kontaktbereich eine Relativbewegung, d.h. ein Durchrutschen, zulässt, ist hingegen für eine Abstützung und Führung zwischen dem ersten und zweiten Werkzeugsegment vorgesehen und leitet so die Kräfte in Längsrichtung zwischen den Werkzeugsegmenten weiter.

In dem in Figur 2 veranschaulichten Ausführungsbeispiel werden jedoch mehr Funktionsflächenpaare eingesetzt, um einen möglichst definierten Bewegungsablauf zwischen dem ersten Werkzeugsegment 10 und dem zweiten Werkzeugsegment 20 zu erreichen.

Das Funktionsflächenpaar, welches zwischen sich eine Relativbewegung zulässt, wird durch die Funktionsflächen 15 und 25 ausgebildet. Wie oben beschrieben, wird das in Eingriff befindliche Funktionsflächenpaar durch die Funktionsflächen 18 und 28 ausgebildet. Dabei befinden sich die Funktionsflächen 18 und 28 in einem formschlüssigen Eingriff, der im Allgemeinen eine bessere Drehmomentübertragung gewährleistet als ein reibschlüssiger Eingriff. Weiterhin befindet sich der Wirk- bzw. Teilkreisdurchmesser der im Eingriff befindlichen Funktionsflächen 18 und 28 in etwa auf der Höhe des Schnittpunktes der Längsachse L1 des ersten Werkzeugsegments 10 und der Längsachse L2 des zweiten Werkzeugsegments 20.

Bei dem Wirkkreisdurchmesser bzw. Teilkreisdurchmesser einer Funktionsfläche handelt es sich um den Durchmesser, auf dem die Kontaktpunkte liegen, wo sich die Umfänge von zwei zusammenwirkenden Funktionsflächen berühren. Wie in der Getriebetechnik kann durch das Verhältnis zwischen den Wirkkreisdurchmessern zweier Getriebeelemente, zum Beispiel zweier Zahnräder, ein Übersetzungsverhältnis bestimmt werden. Da sich die Wirkkreisdurchmesser der Funktionsflächen 18 und 28 auf Höhe des Schnittpunktes zwischen den Längsachsen L1 und L2 befinden und die Längsachse L1 den Mittelpunkt des Wirkkreisdurchmessers der Funktionsfläche 18 sowie die Längsachse L2 den Mittelpunkt des Wirkkreisdurchmessers der Funktionsfläche 28 ausbilden, sind die beiden Wirkkreisdurchmesser in etwa gleich groß, sodass das Übersetzungsverhältnis in etwa 1:1 beträgt.

Weiterhin ist in dem in der Figur 2 dargestellten Ausführungsbeispiel ein Funktionsflächenpaar vorgesehen, das den Einführabschnitt 24 in der Aufnahme 14 unterstützt. Dabei handelt es sich um die radial nach innen gerichtete zweite Funktionsfläche 16 des ersten Werkzeugsegments 10 und die radial nach außen gerichtete zweite Funktionsfläche 26 des zweiten Werkzeugsegments 20. Alternativ oder ergänzend kann das Funktionsflächenpaar, das die radial nach innen gerichtete dritte Funktionsfläche 17 des ersten Werkzeugsegments 10 und die radial nach außen gerichtete dritte Funktionsfläche 27 des zweiten Werkzeugsegments 20 aufweist, als unterstützendes Funktionsflächenpaar vorgesehen sein.

Die Flächen des unterstützenden und führenden Funktionsflächenpaars rollen aufeinander ab. Folglich lassen sie zwischen sich im Wesentlichen keine Relativbewegung zu und sind auch nicht für eine Übertragung eines Drehmoments vorgesehen.

Die Interaktion zwischen den drei Funktionsflächenpaaren wird bei dem vorliegenden Ausführungsbeispiel durch ein Abstützen der Stützfläche 23 auf der Haltefläche 13 in der Aufnahme 14 ergänzt. Insbesondere sorgt die Abstützung der Stützfläche 23 auf der Haltefläche 13 für einen definierten Kontakt zwischen den Funktionsflächen 15 und 25 und den Funktionsflächen 26 und 16. Gleichzeitig wird der Einführabschnitts 24 des zweiten Werkzeugsegments 20 in der Aufnahme 14 des ersten Werkzeugsegments 10 zuverlässig gehalten.

Für ein Einführen des Einführabschnitts 24 in die Aufnahme 14, sodass die Stützfläche 23 des zweiten Werkzeugsegments 20 in proximaler Richtung hinter die Haltefläche 13 des ersten Werkzeugsegments 10 gelangen kann, wird vorzugsweise ein Abschnitt des Einführabschnitts 24 und/oder der radial nach außen gerichteten Funktionsfläche 15 elastisch nachgiebig ausgeführt. Alternativ oder ergänzend kann das Einführen des Einführabschnitts 24 dadurch erfolgen, dass das Werkzeugsegment 30 erst dann vollständig in das erste Werkzeugsegment 10 eingeschraubt wird, nachdem der Einführabschnitt 24 des zweiten Werkzeugsegments 20 durch die distale Öffnung der Aufnahme 14 eingeschoben worden ist.

Im einfachsten Fall sind allerdings der Einführabschnitt 24 und die Aufnahme 14 so dimensioniert, dass ein Einführen des Einführabschnitts 24 ohne speziell dafür vorgesehene elastische Nachgiebigkeit oder Verriegelung durch den Vorsprung 11 des dritten Werkzeugsegments möglich ist. Hierbei beugen die Haltefläche 13 und die Stützfläche 23 im Wesentlichen einem unbeabsichtigten Herausfallen des zweiten Werkzeugsegments 20 aus dem ersten Werkzeugsegment 10 vor, allerdings nicht mit der gleichen Sicherheit, wie die zuvor beschriebenen beiden Ausführungsformen. Dafür ist bei dieser Ausführungsform ein Wechsel des ersten Werkzeugsegments 10 besonders einfach möglich.

Der Antrieb des Werkzeugsegments 20 erfolgt über das in Figur 1 gezeigte Einspannelement 36. Die hierüber eingeleitete Drehung wird direkt auf das erste Werkzeugsegment 10 übertragen. Die Drehmomentübertragung vom ersten Werkzeugsegment 10 zu dem zweiten Werkzeugsegment 20 erfolgt anschließend, wie oben beschrieben, über den in die Aufnahme 14 eingeführten Einführabschnitt 24 auf Höhe des Funktionsflächenpaars 18, 28. Der kinematische Bewegungsablauf wird jedoch maßgeblich durch das Funktionsflächenpaar 15, 25 sowie das Funktionsflächenpaar 16, 26 und/oder das Funktionsflächenpaar 17, 27 bestimmt.

Wie oben beschrieben, ergibt sich der Pfad des Werkzeugs 1 durch das Knochengewebe aufgrund einer Vorbohrung, einer Knochendichteverteilung im Knochengewebe und/oder eines Markraums des zu behandelnden Knochens. Dabei sucht sich das Werkzeugsegment 20 im Allgemeinen den Weg des geringsten Widerstands. Dieser Weg des geringsten Widerstands führt somit das Werkzeugsegment 20.

Wird zunächst nur das Werkzeugsegment 20 geführt, zum Beispiel wenn sich nur dieses im Knochengewebe befindet, ergeben sich zwei oben bereits beschriebene Bewegungsmöglichkeiten zwischen dem ersten Werkzeugsegment 10 und dem zweiten Werkzeugsegment 20. Zum einen kann sich der Einführabschnitts 24 mit der Längsachse L2 auf einer Doppelkegel-Kreisbahn relativ um die Längsachse L1 des ersten Werkzeugsegments 10 bewegen. Gleichzeitig kann in diesem Zustand eine Drehung von dem ersten Werkzeugsegment 10 zu dem zweiten Werkzeugsegment 20 übertragen werden.

Die Bewegung des Einführabschnitts 24 auf einer Doppelkegel-Kreisbahn relativ um die Längsachse L1 des ersten Werkzeugsegments 10 ergibt sich unter Bezugnahme auf die Figur 2 durch den mittels der Funktionsfläche 15 und der Funktionsfläche 16 vorgegebenen kreisförmigen Werkzeugpfad. Alternativ zu einem kreisförmigen Werkzeugpfad sind auch andere Werkzeugpfade denkbar, wie zum Beispiel ein Werkzeugpfad, der aufgrund einer entsprechend ausgebildeten Geometrie der Funktionsfläche 16 und der Funktionsfläche 15 wellenförmig um die Längsachse L1 verläuft. Bei so einer Ausführungsform verändert sich der Winkel α zwischen der Längsachse L1 und L2 in Abhängigkeit der Winkelposition der Längsachse L1 zur Längsachse L2.

Wenn das in Figur 2 dargestellte erste Werkzeugsegment 10 geführt wird, wie zum Beispiel durch die Hand eines Chirurgen, bleiben das erste Werkzeugsegment 10 und das zweite Werkzeugsegment 20 relativ zu einem globalen Koordinatensystem in der gleichen Stellung, wie zum Beispiel der in den Figuren 2 oder 3 dargestellten Stellung. Der Winkel α der Werkzeugsegmente 10 und 20 zueinander ergibt sich aus dem Winkel zwischen den beiden Längsachsen L1 und L2, welcher wiederum durch den Kegelwinkel der Funktionsflächen 15 und 25 bestimmt wird.

Im Folgenden werden einige mögliche Abwandlungsmöglichkeiten der in den Figuren 2 und 3 gezeigten Ausführungsform beschrieben.

In der am einfachsten aufgebauten Ausführungsform erfolgt die Übertragung einer Drehung und Abstützung zwischen den Funktionsflächen 15, 25 und 16, und zwar zum einen an einer Stelle, bei der sich die Funktionsflächen 15 und 25 berühren, und zum anderen an einer dieser Stelle in etwa diametral gegenüberliegenden Stelle, bei der sich die Funktionsflächen 25 und 16 berühren.

Bei dieser Ausführungsform müssen die Funktionsflächen geometrisch entsprechend angepasst sein. Beispielsweise kann sich die radial nach innen gerichtete zweite Funktionsfläche 16 des ersten Werkzeugsegments 10 nach proximal verjüngen. Auch bei dieser Ausführungsform gibt es ein Funktionsflächenpaar, das zwischen sich eine Relativbewegung zulässt, und ein Funktionsflächenpaar, über das die Übertragung eines Drehmoments ermöglicht wird. Jedoch überschneiden sich die Funktionsflächenpaare bei dieser Ausführungsform.

Ist das Funktionsflächenpaar, das zwischen sich eine Relativbewegung zulässt, das Funktionsflächenpaar aus der relativ zu der Längsachse L1 radial nach außen gerichteten ersten Funktionsfläche 15 und der relativ zu der Längsachse L2 radial nach außen gerichteten ersten Funktionsfläche 25, so ist das Funktionsflächenpaar, welches eine Übertragung einer Drehung bzw. eines Drehmoments ermöglicht, zwischen der zweiten Funktionsfläche 16 und der ersten Funktionsfläche 25. In diesem Fall drehen sich das erste Werkzeugsegment 10 und das zweite Werkzeugsegment 20 in der gleichen Richtung.

Je nach Wahl der jeweiligen Wirkkreisdurchmesser der Funktionsfläche 25 und der Funktionsfläche 16 ist es möglich, dass sich das zweite Werkzeugsegment 20 mit einer um das Verhältnis zwischen den Wirkkreisdurchmessern unterschiedlichen Geschwindigkeit zu dem Werkzeugsegment 10 dreht. Neben des einfachen Aufbaus ist somit bei dieser Ausführungsform eine Erhöhung der Drehzahl von dem ersten Werkzeugsegment 10 zu dem zweiten Werkzeugsegment 20 möglich.

Bei einer weiteren alternativen Ausführungsform kann die Drehmomentübertragung zwischen den Funktionsflächen 15 und 25 erfolgen, während die Relativbewegung zwischen den Funktionsflächen 16 und 25 zugelassen wird. Auch hier hängt die Drehgeschwindigkeit zwischen den beiden Werkzeugsegmenten 10 und 20 von dem Verhältnis der Wirkkreisdurchmesser der beiden Funktionsflächen 15 und 25 ab. Hinzu kommt jedoch, dass sich bei dieser Ausführungsform das zweite Werkzeugsegment 20 im Verhältnis zum ersten Werkzeugsegment 10 in entgegengesetzter Richtung dreht. Folglich kommt bei dieser Ausführungsform neben einer Übersetzung eine Drehrichtungsumkehr hinzu.

Um ein stabileres kinematisches Verhalten der Werkzeugsegmente 10 und 20 zueinander zu erreichen, können zwei getrennte Funktionsflächenpaare vorgesehen sein. Übertragen auf die Figur 2 könnte das erste Funktionsflächenpaar aus den Funktionsflächen 15 und 25 gebildet werden und das zweite Funktionsflächenpaar aus den Funktionsflächen 16 und 26. Alternativ könnte auch eines der verbleibenden Funktionsflächenpaare 17 und 27 oder 18 und 28 verwendet werden.

Bei dieser Ausführungsform findet beispielsweise die Relativbewegung zwischen dem Funktionsflächenpaar 15, 25 statt, während die Übertragung der Drehbewegung durch einen Eingriff zwischen dem Funktionsflächenpaar 16, 26 erfolgt. Wie oben beschrieben, drehen sich in diesem Fall das Werkzeugsegment 10 und das Werkzeugsegment 20 in derselben Richtung, wobei die Winkelgeschwindigkeiten des Werkzeugsegments 10 zum Werkzeugsegment 20 von dem Verhältnis der Wirkkreisdurchmesser der Funktionsfläche 26 zur Funktionsfläche 16 abhängt.

Im umgekehrten Fall, d.h. einem Eingriff zwischen den Funktionsflächen 15 und 25 sowie eine Relativbewegung zwischen den Funktionsflächen 16 und 26, ist der Drehsinn zwischen den Werkzeugsegmenten 10 und 20 entgegengesetzt zu dem der vorangehenden Ausführungsform. Für die Drehgeschwindigkeit gilt wiederum das Verhältnis der Wirkkreisdurchmesser zueinander.

Auch bei den beschriebenen Abwandlungen wird bevorzugt an dem ersten Werkzeugsegment eine Haltefläche 13 und an dem zweiten Werkzeugsegment eine Stützfläche 23 vorgesehen.

Weiterhin liegt bei den Abwandlungen ein Unterschied bei den Drehgeschwindigkeiten der beiden Werkzeugsegmente vor, da die Drehmomentübertragung abweichend von dem Ausführungsbeispiel in Figur 2 nicht auf der Höhe des Schnittpunktes zwischen der Längsachse L1 des ersten Werkzeugsegments 10 und der Längsachse L2 des zweiten Werkzeugsegments 20 liegt. Folglich unterscheiden sich die Wirkkreisdurchmesser des Einführabschnitts 24 und der Aufnahme 14 bzw. des dritten Werkzeugabschnitts 30 voneinander.

Weiterhin können auch mehr als zwei Werkzeugsegmente erfindungsgemäß in einem Winkel zueinander stehen. Beispielsweise kann das dritte Werkzeugsegment ebenfalls in einem Winkel zu dem ersten Werkzeugsegment stehen und so eine für ein Implantat vorbestimmte Aussparung mit zwei Winkeln ausbilden.

Das vorliegende Werkzeug ist insbesondere für Implantate vorgesehen, die in Röhrenknochen implantiert werden. Hierzu gehören insbesondere Hüftimplantate, da hier die Eigenschaft des Werkzeugs, ein mit einem Winkel vorgesehenes Loch vorbereiten zu können, im Hinblick auf den gekrümmten Verlauf des natürlichen Schenkelhalses von besonderem Vorteil ist.

### BEZUGSZEICHENLISTE

- 1: Werkzeug
- 10: erstes Werkzeugsegment
- 11: Vorsprung in der Aufnahme
- 12: Schneidelement
- 13: Haltefläche in der Aufnahme
- 14: Aufnahme
- 15: radial nach außen gerichtete (erste) Funktionsfläche des ersten Werkzeugsegments
- 16: radial nach innen gerichtete (zweite) Funktionsfläche des ersten Werkzeugsegments
- 17: radial nach innen gerichtete (dritte) Funktionsfläche des ersten Werkzeugsegments
- 18: radial nach innen gerichtete (vierte) Funktionsfläche des ersten Werkzeugsegments
- 19: Gewindeloch
- 20: zweites Werkzeugsegment
- 21: distale Basis des von der Funktionsfläche 26 gebildeten Kegelstumpfs
- 22: Schneidelement
- 23: Stützfläche
- 24: Einführabschnitt
- 25: radial nach außen gerichtete (erste) Funktionsfläche des zweiten Werkzeugsegments
- 26: radial nach außen gerichtete (zweite) Funktionsfläche des zweiten Werkzeugsegments
- 27: radial nach außen gerichtete (dritte) Funktionsfläche des zweiten Werkzeugsegments
- 28: radial nach außen gerichtete (vierte) Funktionsfläche des zweiten Werkzeugsegments
- 29: Endpunkt des Einführabschnitts
- 30: drittes Werkzeugsegment
- 32: Schneidelement
- 34: Gewindeabschnitt
- 36: Einspannelement
- I16: Innenabmessung der zweiten Funktionsfläche des ersten Werkzeugsegments
- I17: Innenabmessung der dritten Funktionsfläche des ersten Werkzeugsegments
- I18: Innenabmessung der vierten Funktionsfläche des ersten Werkzeugsegments
- L1: erste Längsachse
- L2: zweite Längsachse
- α: Winkel zwischen den Längsachsen L1 und L2

## Patentansprüche

1. Werkzeug (1) zum Erzeugen einer Aussparung in Knochengewebe für ein Implantat, wobei das Werkzeug aufweist:
ein erstes längliches Werkzeugsegment (10) mit einer ersten Längsachse (L1) und einer Aufnahme (14), wobei die Aufnahme um die erste Längsachse eine im Wesentlichen radial nach außen gerichtete erste Funktionsfläche (15) und eine radial nach innen gerichtete zweite Funktionsfläche (16, 17, 18) aufweist,
ein zweites längliches Werkzeugsegment (20) mit einer zweiten Längsachse (L2) und einem Einführabschnitt (24), der in die Aufnahme des ersten Werkzeugsegments einführbar ist, wobei der Einführabschnitt um die zweite Längsachse mindestens eine radial nach außen gerichtete Funktionsfläche (25, 26, 27, 28) aufweist, die mit der nach außen gerichteten ersten Funktionsfläche (15) des ersten Werkzeugsegments zusammenwirkt, und eine der mindestens einen Funktionsfläche (25, 26, 27, 28) des Einführabschnitts mit der zweiten Funktionsfläche (16, 17, 18) des ersten Werkzeugsegments zusammenwirkt,
wobei sich die erste Längsachse und die zweite Längsachse schneiden, eines der zusammenwirkenden Funktionsflächenpaare (15, 16, 17, 18, 25, 26, 27, 28) für die Übertragung eines Drehmoments miteinander im Eingriff steht und das andere der zusammenwirkenden Funktionsflächenpaare (15, 16, 17, 18, 25, 26, 27, 28) zwischen sich eine Relativbewegung zulässt.

2. Werkzeug (1) nach Anspruch 1, bei dem die erste Funktionsfläche (15) des ersten Werkzeugsegments (10) durch einen Vorsprung (11) in der Aufnahme (14) ausgebildet wird, wobei der Vorsprung vorzugsweise konisch oder zylindrisch ist.

3. Werkzeug (1) nach einem der vorstehenden Ansprüche, bei dem das in Eingriff stehende Funktionsflächenpaar (15, 16, 17, 18, 25, 26, 27, 28) für die Drehmomentübertragung formschlüssig im Eingriff ist.

4. Werkzeug (1) nach einem der vorstehenden Ansprüche, bei dem zumindest die Querschnitte der Funktionsflächen (15, 16, 17, 18, 25, 26, 27, 28) von mindestens einem Funktionsflächenpaar senkrecht zu der jeweiligen Längsachse (L1, L2) einen im Wesentlichen kreisförmigen Umfang aufweisen.

5. Werkzeug nach einem der vorstehenden Ansprüche, bei dem das erste Werkzeugsegment (10) und das zweite Werkzeugsegment (20) mindestens zwei getrennte zwischen ihnen zusammenwirkende Funktionsflächenpaare (15, 16, 17, 18, 25, 26, 27, 28) aufweisen

6. Werkzeug nach einem der Ansprüche 1 bis 4, bei dem das erste Werkzeugsegment (10) und das zweite Werkzeugsegment (20) mindestens drei getrennte, zwischen ihnen zusammenwirkende Funktionsflächenpaare (15, 16, 17, 18, 25, 26, 27, 28) aufweisen, wobei das erste Funktionsflächenpaar (15, 25) zwischen sich eine Relativbewegung zulässt, das zweite Funktionsflächenpaar (16, 26) den Einführabschnitt (24) in der Aufnahme (14) unterstützt und das Drehmoment zwischen dem ersten und dem zweiten Werkzeugsegment durch das dritte Funktionsflächenpaar (18, 28) übertragbar ist.

7. Werkzeug nach einem der vorstehenden Ansprüche, bei dem die radial nach außen gerichtete Funktionsfläche (15) des ersten Werkzeugsegments (10) auswechselbar und vorzugsweise ein Teil eines dritten Werkzeugsegments (30) ist.

8. Werkzeug nach einem der vorstehenden Ansprüche, bei dem mindestens eines der Werkzeugsegmente (10, 20, 30) an einer nach außen gewandten Umfangsfläche mindestens ein Schneidelement (12, 22, 32) aufweist.

9. Werkzeug nach einem der vorstehenden Ansprüche, bei dem die Aufnahme (14) des ersten Werkzeugsegments (10) eine Haltefläche (13) aufweist, die durch eine Querschnittserweiterung innerhalb der Aufnahme ausgebildet ist und an der sich eine dazu korrespondierende Querschnittserweiterung des Einführabschnitts (24) im eingeführten Zustand abstützt, sodass das zweite Werkzeugsegment im ersten Werkzeugsegment gehalten wird.

10. Werkzeug (1) nach einem der vorstehenden Ansprüche, bei dem auf der Höhe des Schnittpunkts der ersten Längsachse (L1) und der zweiten Längsachse (L2) ein Funktionsflächenpaar angeordnet ist, das eine radial nach außen gerichtete Funktionsfläche (26, 27, 28) des Einführabschnitts (24) und eine radial nach innen gerichtete Funktionsfläche (16, 17, 18) der Aufnahme (14) aufweist und das Funktionsflächenpaar bevorzugt für die Drehmomentübertragung geeignet ist.

11. Werkzeugsegment (10) mit einer Längsachse (L1), um die zum Einführen eines weiteren Werkzeugsegments (20) eine Aufnahme ausgebildet ist, die eine Öffnung, eine radial nach innen gerichteten Funktionsfläche (16, 17, 18) und eine Endfläche, die zumindest abschnittsweise durch eine radial nach außen gerichtete Funktionsfläche (15) ausgebildet ist, aufweist, wobei eine der Funktionsflächen (15, 16, 17, 18) für die Übertragung eines Drehmoments zu dem weiteren Werkzeugsegment vorgesehen ist und die andere Funktionsfläche (15, 16, 17, 18) eingerichtet ist, eine Relativbewegung zu dem weiteren Werkzeugsegment zuzulassen.

12. Satz aus einem Werkzeug nach einem der Ansprüche 1 bis 10 und einem Implantat, insbesondere einer Endoprothese, das in eine mit dem Werkzeug im Knochengewebe eines Röhrenknochens erzeugbare Aussparung einsetzbar ist.
